(19) Europäisches Patentamt
European Patent Office
Office européen des brevets

(11) EP 0 998 919 A2

(12) **EUROPÄISCHE PATENTANMELDUNG**

(43) Veröffentlichungstag:
**10.05.2000 Patentblatt 2000/19**

(51) Int. Cl.$^7$: **A61K 9/20**

(21) Anmeldenummer: **99118792.3**

(22) Anmeldetag: **23.09.1999**

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(30) Priorität: **24.09.1998 DE 19843903**

(71) Anmelder:
**BASF AKTIENGESELLSCHAFT**
**67056 Ludwigshafen (DE)**

(72) Erfinder:
- **Kothrade, Stephan**
  **67117 Limburgerhof (DE)**
- **Berndl, Gunther**
  **67273 Herxheim (DE)**
- **Sanner, Axel**
  **67227 Frankenthal (DE)**
- **Simon, Dirk**
  **67112 Mutterstadt (DE)**

(74) Vertreter:
**Kinzebach, Werner, Dr. et al**
**Patentanwälte,**
**Reitstötter, Kinzebach & Partner,**
**Ludwigsplatz 4**
**67059 Ludwigshafen (DE)**

(54) **Feste Dosierungsform mit copolymerem Bindemittel**

(57) Die Erfindung betrifft feste Dosierungsformen, enthaltend wenigstens ein polymeres Bindemittel und wenigstens einen Wirkstoff und Verfahren zur Herstellung der festen Dosierungsformen. Als polymeres Bindemittel verwendet man ein Copolymer eines N-Vinyllactams mit Methylmethacrylat , welches eine Wasseraufnahme durch Quellen von weniger als 50 Gew.-%, bezogen auf das Gewicht des Bindemittels, aufweist. Die festen Dosierungsformen setzen den Wirkstoff langsam frei.

EP 0 998 919 A2

## Beschreibung

Die Erfindung betrifft feste Dosierungsformen, enthaltend wenigstens ein polymeres Bindemittel und wenigstens einen Wirkstoff und gegebenenfalls übliche Additive und ein Verfahren zur Herstellung der festen Dosierungsformen.

Feste Dosierungsformen, die physiologisch verträgliche Polymere als Bindemittel enthalten, gewinnen zunehmend an Bedeutung, insbesondere für Dosierungsformen mit verzögerter Wirkstoffabgabe. So beschreibt die EP-A-609 961 feste Dosierungsformen auf Basis physiologisch verträglicher Copolymere, die zum Erreichen einer gleichmäßigen Wirkstoffabgabe einen Kern/Schale-Aufbau aus verschiedenen Copolymerisaten aufweisen.

Die US-A-3,900,559 beschreibt Wirkstoffzusammensetzungen mit verzögerter Wirkstoffabgabe auf Basis von vernetzten Copolymeren, die unter anderem N-Vinyllactame und (Meth)acrylate, insbesondere (Meth)acrylate von Di- und Polyolen, als Comonomere enthalten. Dabei erfolgt die Einbringung des Wirkstoffs vorzugsweise durch Tränken des Polymers mit einer Lösung des Wirkstoffs bzw. durch Polymerisation der Monomere in Gegenwart des gelösten Wirkstoffs.

Um eine verzögerte Wirkstoffabgabe zu erreichen, werden in der US-A-4,248,855 die als Bindemittel eingesetzten, Säuregruppen enthaltenden Polymerisate gelöst und mit einer Lösung eines salzbildenden Wirkstoffs versetzt und das Wirkstoff/Polymer-Salz für die pharmazeutische Anwendung formuliert.

In der WO 89/06957 werden pharmazeutische Formulierungen mit kontrollierter Wirkstofffreisetzung beschrieben, bei denen das als Bindemittel eingesetzte N-Vinylpyrrolidon/Alkyl (meth) acrylat-Copolymerisat auf Grund seines hohen Quellvermögens von 50 % bis 250 % eine verzögerte Wirkstoffabgabe ermöglicht.

Die genannten festen Dosierungsformen des Standes der Technik haben den Nachteil, dass die Einbringung des Wirkstoffes und/oder seine Freisetzung durch Diffusion erfolgen. Dabei ist die Freisetzung an das hohe Quellvermögen des Bindemittels gekoppelt und damit von dem Diffusionsverhalten des jeweiligen Wirkstoffes im jeweiligen Bindemittel abhängig. Die Einbringung des Wirkstoffs und/oder die Herstellung der festen Dosierungsform erfordert aufwendige oder mehrstufige Verfahren, um die gewünschte verzögerte Freisetzung zu erreichen. Zudem werden die klassischen Verfahren zur Herstellung fester pharmazeutischer Dosierungsformen, insbesondere Tabletten, diskontinuierlich durchgeführt und umfassen mehrere Stufen.

Ein wesentlich einfacheres kontinuierliches Verfahren zur Herstellung fester pharmazeutischer Dosierungsformen, bei dem man eine wirkstoffhaltige, lösungsmittelfreie Schmelze aus einem polymeren wirkstoffhaltigen Bindemittel extrudiert und den extrudierten Strang zu der gewünschten Dosierungsform formt, beispielsweise in einem Kalander mit Formwalzen, ist aus der EP-A-240 904, EP-A-240 906, EP-A-337 256 und der EP-A-358 105 (Schmelzextrusion) bekannt.

In der EP-A-240 904 werden als polymere Bindemittel Polyvinylpyrrolidon oder Copolymerisate von N-Vinylpyrrolidon mit unter anderem Vinylestern, ungesättigten Carbonsäuren, darunter auch Acrylsäure und Methacrylsäure, ihren Amiden und ihren Estern mit $C_1$-$C_{12}$-Alkanolen genannt, aber in den Beispielen nur Vinylpyrrolidon/Vinylacetat-Copolymerisate eingesetzt. Die N-Vinylpyrrolidon/Vinylacetat-Copolymerisate besitzen, ebenso wie die in der DE 197 53 300.0 offenbarten N-Vinylcaprolactam-Polymerisate und -Copolymerisate, ein sehr günstiges Eigenschaftsprofil als Bindemittel für die Herstellung fester Dosierungsformen durch Pressen unter Druck und Temperatur und insbesondere durch Schmelzextrusion, jedoch haben sie den Nachteil, dass sie den Wirkstoff relativ rasch freisetzen.

Der vorliegenden Erfindung liegt daher die Aufgabe zu Grunde, feste Dosierungsformen zur Verfügung zu stellen, die eine langsame Freisetzung des Wirkstoffes auch über längere Zeiträume ermöglichen und sich einfach und kostengünstig, z. B. durch Schmelzextrusion, herstellen lassen.

Überraschenderweise wurde nun gefunden, dass diese Aufgabe gelöst wird, wenn man als Bindemittel ein Polymerisat aus wenigstens einem N-Vinyllactam, Methylmethacrylat und gegebenenfalls weiteren copolymerisierbaren Monomeren verwendet, das eine Wasseraufnahme durch Quellen von weniger als 50 Gew.-%, bezogen auf das Gewicht des Bindemittels, aufweist.

Gegenstand der vorliegenden Erfindung ist daher eine feste Dosierungsform, enthaltend wenigstens ein polymeres Bindemittel und wenigstens einen Wirkstoff und gegebenenfalls übliche Additive, die dadurch gekennzeichnet ist, dass das polymere Bindemittel

a) 10 bis 60 Gew.-% mindestens eines N-Vinyllactams der Formel I

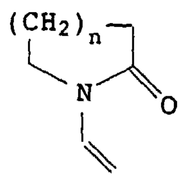

(I)

worin n für 1, 2 oder 3 steht,

b) 40 bis 90 Gew. -% Methylmethacrylat

einpolymerisiert enthält und eine Wasseraufnahme durch Quellen von weniger als 50 Gew.-%, bezogen auf das Gewicht des Bindemittels, aufweist.

Das als Bindemittel verwendete Polymerisat enthält vorzugsweise 20 bis 55 Gew.-% und besonders bevorzugt 20 bis 45 Gew.-% mindestens eines N-Vinyllactams der Formel I einpolymerisiert. Das Bindemittel kann ein Gemisch von N-Vinyllactamen der Formal I einpolymerisiert enthalten, bevorzugt enthält das Bindemittel nur ein N-Vinyllactan einpolymerisiert. Bei den N-Vinyllactamen der Formel I handelt es sich um N-Vinylpyrrolidon, N-Vinylpiperidon und N-Vinylcaprolactam; bevorzugt ist N-Vinylpyrrolidon. Als Comonomer enthält das Bindemittel vorzugsweise 45 bis 80 Gew. -% und besonders bevorzugt 55 bis 80 Gew. -% Methylmethacrylat einpolymerisiert.

Vorzugsweise weist das Bindemittel eine Wasseraufnahme durch Quellen von weniger als 40 %, bezogen auf das Gewicht des Bindemittels, auf. Definiert wird die Wasseraufnahme in Prozent gemäß folgender Formel:

$$\frac{\text{Gew. hydratisiertes Bindemittel - Gew. trockenes Bindemittel}}{\text{Gew. trockenes Bindemittel}} \cdot 100\ \%$$

Bestimmt wurde die Wasseraufnahme an dünnen Filmen des jeweiligen Bindemittels nach 24 Stunden Quellenlassen in Wasser bei Raumtemperatur und Entfernen der oberflächlich anhaftenden Wassertropfen.

Darüber hinaus kann das polymere Bindemittel mindestens ein weiteres copolymerisierbares Monomer (weiteres Comonomer) in einer Menge von 0,1 bis 50 Gew.-%, vorzugsweise 0,5 bis 40 Gew.-% und besonders bevorzugt 0,5 bis 30 Gew.-%, bezogen auf das Gesamtgewicht der Monomeren, einpolymerisiert enthalten. Geeignete weitere Comonomere sind insbesondere Vinylester aliphatischer $C_1$-$C_{22}$-Carbonsäuren, wie Vinylformiat, Vinylacetat, Vinylpropionat, Vinylbutyrat, Vinylvalerat, Vinylcapronat, Vinyloctanoat, Vinylcaprat, Vinyllaurat, Vinylmyristat und Vinylpalmitat, wobei Vinylacetat besonders bevorzugt ist, monoethylenisch ungesättigte Carbonsäuren mit 3 bis 8 Kohlenstoffatomen, wie Acrylsäure, Methacrylsäure, Dimethacrylsäure, Ethacrylsäure, Maleinsäure, Citraconsäure, Methylenmalonsäure, Allylessigsäure, Vinylessigsäure, Crotonsäure, Fumarsäure, Mesaconsäure und Itaconsäure sowie die Halbester der erwähnten Dicarbonsäuren mit $C_1$- bis $C_8$-Alkanolen. Bevorzugt sind Acrylsäure, Methacrylsäure, Maleinsäure oder Mischungen davon. Die monoethylenisch ungesättigten Carbonsäuren können in Form der freien Säure, der Anhydride oder in partiell oder vollständig neutralisierter Form eingesetzt werden. Zur Neutralisation verwendet man vorzugsweise Alkalimetall- oder Erdalkalimetallbasen, Ammoniak oder Amine, z. B. Natriumhydroxid, Kaliumhydroxid, Natriumcarbonat, Kaliumcarbonat, Natriumhydrogencarbonat, Magnesiumoxid, Calciumhydroxid, Calciumoxid, gasförmiges oder wässriges Ammoniak, Triethylamin, Ethanolamin, Diethanolamin, Morpholin, Diethylentriamin oder Tetraethylenpentamin. Die erwähnten monoethylenisch ungesättigten Carbonsäuren mit 3 bis 8 C-Atomen sind vorzugsweise zu ≤ 10 Gew.-% und insbesondere ≤ 5 Gew.-% enthalten.

Weiterhin eignen sich als weitere Comonomere beispielsweise die Ester der oben angegebenen Carbonsäuren mit $C_1$- bis $C_8$-Alkanolen, $C_1$- bis $C_4$-Diolen, Mono- und Di-$C_1$ bis $C_4$-alkylamino-$C_1$- bis $C_4$-alkanolen sowie die Amide, Mono- und Di-$C_1$- bis $C_4$-alkylamide und Nitrile dieser Carbonsäuren, z. B. Acrylsäuremethylester, Acrylsäureethylester, Acrylsäurepropylester, Acrylsäurebutylester, 2-Ethylhexylacrylat, Methacrylsäureethylester, Methacrylsäurepropylester, Methacrylsäurebutylester, 2-Ethylhexylmethacrylat, Hydroxyethylacrylat, Hydroxypropylacrylat, Hydroxybutylacrylat, Hydroxyisobutylacrylat, Hydroxyethylmethacrylat, Hydroxypropylmethacrylat, Hydroxybutylmethacrylat, Hydroxyisobutylmethacrylat, Maleinsäuremonomethylester, Maleinsäuredimethylester, Maleinsäuremonoethylester, Maleinsäurediethylester, Maleinsäuremonobutylester, Maleinsäuredibutylester, Acrylamid, Methacrylamid, N,N-Dimethylacrylamid, N-tert. -Butylacrylamid, Acrylnitril, Methacrylnitril, Dimethylaminoethylacrylat, Diethylaminoethylacrylat, Diethylaminoethylmethacrylat sowie die Salze der zuletzt genannten Monomeren mit Carbonsäuren oder Mineralsäuren oder die quaternisierten Produkte.

Außerdem eignen sich als copolymerisierbare weitere Monomere Acrylamidoglykolsäure, Vinylsulfonsäure, Allylsulfonsäure, Methallyltsulfonsäure, Styrolsulfonsäure, Acrylsäure-( 3-sulfopropyl)ester, Methacrylsäure-(3-sulfopropyl)ester und Acrylamidomethylpropansulfonsäure sowie Phosphonsäuregruppen enthaltende Monomere wie Vinylphosphonsäure, Allylphosphonsäure und Acrylamidomethylpropanphosphonsäure. Weitere geeignete copolymerisierbare Monomere sind N-Vinylimidazol, N-Vinyl-2-methylimidazol, N-Vinyl-4-methylimidazol, sowie Vinylaromaten, wie Styrol. Es können selbstverständlich auch Mischungen der genannten Monomere eingesetzt werden.

Gegebenenfalls kann das polymere Bindemittel zusätzlich 0,1 bis 2,0 Gew.-% mindestens eines vernetzenden Monomers einpolymerisiert enthalten. Unter vernetzenden Monomeren sind hier im Wesentlichen di-, tri- und polyolefinische Monomere und Macromere zu verstehen. Geeignete vernetzende Monomere umfassen z. B. die Di-, Tri- oder Polyester ethylenisch ungesättigter Carbonsäuren mit niedermolekularen, zwei-, drei- oder mehrwertigen Alkoholen, vorzugsweise die Acrylsäure- und Methacrylsäureester von zwei-, drei- und mehrwertigen Alkoholen, wie Ethylenglykol, Propylenglykol und deren höherkondensierte Vertreter, z. B. Diethylenglykol, Triethylenglykol, Dipropylenglykol und Tripropylenglykol, sowie Butandiol, Pentandiol, Hexandiol, Propantriol und Trimethylolpropan. Gleichfalls geeignet sind Polyether(meth)acrylate, Polyester(meth)acrylate und Polyurethan(meth)acrylate, die zwei oder mehr $\alpha,\beta$-ethylenisch ungesättigte Gruppen tragen. Ebenfalls geeignet sind die Di-, Tri- und Polyether der genannten di- und polyfunktionalen Alkohole mit Vinylalkohol, sowie Vinylaromaten, wie z. B. Di- und Trivinylbenzol, Divinylharnstoffe, z.B. Divinylmethylenharnstoff und Diallylamine, wie Diallylamin und Diallylammoniumchlorid. Weitere vernetzende Monomere und Macromere sind dem Fachmann bekannt und beispielsweise in P. K. T. Oldring (Hrsg.), Chemistry and Technology of UV- and EB-Formulations for Coatings and Paints, Vol. II, SITA Technology, London 1991, beschrieben.

In bevorzugten Ausführungsformen enthalten die polymeren Bindemittel im Wesentlichen 30 bis 50 Gew.-% N-Vinylpyrrolidon und 50 bis 70 Gew.-% Methylmethacrylat einpolymerisiert.

Die Herstellung der Copolymerisate erfolgt nach bekannten Verfahren, z. B. der Lösungs-, Fällungs-, Suspensions- oder umgekehrten Suspensionspolymerisation, oder der Emulsions- bzw. umgekehrten Emulsionspolymerisation unter Verwendung von Verbindungen, die unter Polymerisationsbedingungen Radikale bilden.

Die Polymerisationstemperaturen liegen üblicherweise in dem Bereich von 30 bis 200 °C, vorzugsweise 40 bis 110 °C. Geeignete Initiatoren sind beispielsweise Azo- und Peroxyverbindungen sowie die üblichen Redoxinitiatorsysteme, wie Kombinationen aus Wasserstoffperoxid und reduzierend wirkende Verbindungen, z. B. Na-. triumsulfit, Natriumbisulfit, Natriumformaldehydsulfoxylat und Hydrazin.

Die Copolymeren besitzen K-Werte von mindestens 7, vorzugsweise 10 bis 100, besonders bevorzugt 10 bis 50. Die K-Werte werden bestimmt nach H. Fikentscher, Cellulose-Chemie, Band 13, (1932) 58-64 und 71-74, in wässriger Lösung oder in einem organischen Lösungsmittel bei 25 °C, bei Konzentrationen, die je nach K-Wert-Bereich zwischen 0,1 % und 5 % liegen.

Neben den oben beschriebenen polymeren Bindemitteln können bis zu 95 Gew.-%, insbesondere bis zu 85 Gew.-% und bevorzugt bis zu 75 Gew.-% weitere Bindemittel, bezogen auf das Gesamtgewicht des Bindemittels, eingesetzt werden. Wenn vorhanden, werden im Allgemeinen mindestens 0,1 Gew.-%, vorzugsweise mindestens 0,5 Gew.-%, des weiteren Bindemittels eingesetzt. Geeignet sind Polymere, Copolymere, Cellulosederivate, Stärke und Stärkederivate, beispielsweise:

Polyvinylpyrrolidon (PVP), Copolymerisate von N-Vinylpyrrolidon (NVP) und Vinylacetat oder Vinylpropionat, Copolymerisate von Vinylacetat und Crotonsäure, teilverseiftes Polyvinylacetat, Polyvinylalkohol, Polyhydroxyalkylacrylate, Polyhydroxyalkylmethacrylate, Polyacrylate und Polymethacrylate (Eudragit-Typen), Copolymerisate von Methylmethacrylat und Acrylsäure, Polyacrylamide, Poltyethylenglykole, Polyvinylformamid (gegebenenfalls partiell oder vollständig hydrolysiert), Celluloseester, Celluloseether, insbesondere Methylcellulose und Ethylcellulose, Hydroxyalkylcellulosen, insbesondere Hydroxypropylcellulose, Hydroxyalkyl-Altkylcellulosen, insbesondere Hydroxypropyl-Ethylcellulose, Cellulosephthalate, insbesondere Celluloseacetatphthalat und Hydroxypropylmethylcellulosephthalat, und Mannane, insbesondere Galactomannane. Davon sind Polyvinylpyrrolidon, Copolymerisate von N-Vinylpyrrolidon und Vinylestern, Polyhydroxyalkylacrylate, Polyhydroxyalkylmethacrylate, Polyacrylate, Polymethacrylate, Alkylcellulosen und Hydroxyalkylcellulosen besonders bevorzugt.

Ebenfalls Gegenstand der vorliegenden Erfindung sind feste Dosierungsformen gemäß obiger Beschreibung in Form pharmazeutischen Dosierungsformen, Pflanzenbehandlungsmitteln, Futtermittelzusatzstoffen und Nahrungsmittelzusätzen.

Weiterer Gegenstand der Erfindung ist ein Verfahren zur Herstellung der oben beschriebenen Dosierungsform, das dadurch gekennzeichnet ist, dass man die Komponenten gewünschtenfalls zunächst aufschmilzt oder direkt mischt und das erhaltene plastische Gemisch zur gewünschten Dosierungsform formt.

Die erfindungsgemäßen Bindemittel eignen sich für alle gängigen Verfahren zur Herstellung fester Dosierungsformen, wie Granulieren, Mahlen, Pressen, Formgießen, Tablettieren unter Druck, Tablettieren unter Druck und Wärme und insbesondere für die Extrusion bzw. Schmelzextrusion.

Für den Einsatz in der Schmelzextrusion muss das polymere Bindemittel in der Gesamtmischung aller Komponenten im Bereich von 50 bis 180 °C, vorzugsweise 60 bis 130 °C erweichen oder schmelzen. Die Glasübergangstemperatur der Mischung muss daher unter 180 °C, vorzugsweise unter 130 °C liegen. Erforderlichenfalls wird sie durch übliche, pharmakologisch akzeptable weichmachende Hilfsstoffe herabgesetzt. Die Menge an Weichmacher beträgt

höchstens 30 Gew.-%, bezogen auf das Gesamtgewicht von Bindemittel und Weichmacher, damit lagerstabile Arzneiformen gebildet werden, die keinen kalten Fluss zeigen. Vorzugsweise aber enthält das Gemisch keinen Weichmacher.

Beispiele für derartige Weichmacher sind:

langkettige Alkohole, Ethylenglykol, Propylenglykol, Glycerin, Trimethylolpropan, Triethylenglykol, Butandiole, Pentanole, wie Pentaerythrit, Hexanole, Polyethylenglykole, Polypropylenglykole, Polyethylenpropylenglykole, Silicone, aromatische Carbonsäureester (z. B. Dialkylphthalate, Trimellithsäureester, Benzoesäureester, Terephthalsäureester) oder aliphatische Dicarbonsäureester (z. B. Dialkyladipate, Sebacinsäureester, Azelainsäureester, Zitronen- und Weinsäureester), Fettsäureester, wie Glycerinmono-, Glycerindi- oder Glycerintriacetat oder Natriumdiethylsulfosuccinat. Die Konzentration an Weichmacher beträgt im Allgemeinen 0,5 bis 15, vorzugsweise 0,5 bis 5 Gew.-%, bezogen auf das Gesamtgewicht des Gemisches.

Übliche galenische Hilfsstoffe, deren Gesamtmenge bis zu 100 Gew.-%, bezogen auf das Polymerisat, betragen kann, sind z. B. Streckmittel bzw. Füllstoffe, wie Silikate oder Kieselerde, Magnesiumoxid, Aluminiumoxid, Titanoxid, Stearinsäure oder deren Salze, z. B. das Magnesium- oder Calciumsalz, Methylcellulose, Natrium-Carboxymethylcellulose, Talkum, Saccharose, Lactose, Getreide- oder Maisstärke, Kartoffelmehl, Polyvinylalkohol, insbesondere in einer Konzentration von 0,02 bis 50, vorzugsweise 0,20 bis 20 Gew.-%, bezogen auf das Gesamtgewicht des Gemisches.

Schmiermittel, wie Aluminium- und Calciumstearat, Talkum und Silicone, in einer Konzentration von 0,1 bis 5, vorzugsweise 0,1 bis 3 Gew.-%, bezogen auf das Gesamtgewicht des Gemisches.

Fließmittel, wie tierische oder pflanzliche Fette, insbesondere in hydrierter Form und solche, die bei Raumtemperatur fest sind. Diese Fette haben vorzugsweise einen Schmelzpunkt von 50°C oder höher. Bevorzugt sind Triglyceride der $C_{12}$-, $c_{14}$-, $C_{16}$- und $C_{18}$-Fettsäuren. Auch Wachse, wie Carnaubawachs, sind brauchbar. Diese Fette und Wachse können vorteilhaft alleine oder zusammen mit Mono- und/oder Diglyceriden oder Phosphatiden, insbesondere Lecithin, zugemischt werden. Die Mono- und Diglyceride stammen vorzugsweise von den oben erwähnten Fettsäuretypen ab. Die Gesamtmenge an Fetten, Wachsen, Mono-, Diglyceriden und/oder Lecithinen beträgt 0,1 bis 30, vorzugsweise 0,1 bis 5 Gew.-%, bezogen auf das Gesamtgewicht der Masse für die jeweilige Schicht;

Farbstoffe, wie Azofarbstoffe, organische oder anorganische Pigmente oder Farbstoffe natürlicher Herkunft, wobei anorganische Pigmente in einer Konzentration von 0,001 bis 10, vorzugsweise 0,5 bis 3 Gew.-%, bezogen auf das Gesamtgewicht des Gemisches bevorzugt sind;

Stabilisatoren, wie Antioxidanzien, Lichtstabilisatoren, Hydroperoxid-Vernichter, Radikalfänger, Stabilisatoren gegen mikrobiellen Befall.

Ferner können Netz-, Konservierungs-, Spreng-, Adsorptions-, Formentrenn- und Treibmittel zugesetzt werden (vgl. z. B. H. Sucker et al., Pharmazeutische Technologie, Thieme-Verlag, Stuttgart 1978).

Unter Hilfsstoffen im Sinne der Erfindung sind auch Substanzen zur Herstellung einer festen Lösung des Wirkstoffs zu verstehen. Diese Hilfsstoffe sind beispielsweise Pentaerythrit und Pentaerythrit-tetraacetat, Polymere wie z. B. Polyethylen- bzw. Polypropylenoxide und deren Blockcopolymere (Poloxamere), Phosphatide wie Lecithin, Homo- und copolymere des Vinylpyrrolidons, Tenside wie Polyoxyethylen-40-stearat sowie Zitronen- und Bernsteinsäure, Gallensäuren, Sterine und andere wie z. B. bei J. L. Ford, Pharm. Acta Helv. 61, 69-88 (1986) angegeben.

Als Hilfsstoffe gelten auch Zusätze von Basen und Säuren zur Steuerung der Löslichkeit eines Wirkstoffes (siehe beispielsweise K. Thoma et al., Pharm. Ind. 51, 98-101 (1989)).

Einzige Voraussetzung für die Eignung von Hilfsstoffen für die Schmelzextrusion ist eine ausreichende Temperaturstabilität.

Unter Wirkstoffen im Sinne der Erfindung sind alle Stoffe mit einer physiologischen Wirkung zu verstehen, sofern sie sich unter den Verarbeitungsbedingungen nicht zersetzen. Es handelt sich insbesondere um pharmazeutische Wirkstoffe (für Mensch und Tier), wirkstoffe für die Pflanzenbehandlung, Insektizide, Futter- und Nahrungsmittelwirkstoffe, Riechstoffe und Parfümöle. Die Wirkstoffmenge pro Dosiseinheit und die Konzentration können je nach Wirksamkeit und Freisetzungsgeschwindigkeit in weiten Grenzen variieren. Die einzige Bedingung ist, dass sie zur Erzielung der gewünschten Wirkung ausreichen. So kann die Wirkstoffkonzentration im Bereich von 0,1 bis 95, vorzugsweise von 20 bis 80, insbesondere 30 bis 70 Gew.-% liegen. Auch Wirkstoff-Kombinationen können eingesetzt werden. Wirkstoffe im Sinne der Erfindung sind auch Vitamine und Mineralstoffe. Zu den Vitaminen gehören die Vitamine der A-Gruppe, der B-Gruppe, worunter neben $B_1$, $B_2$, $B_6$ und $B_{12}$ sowie Nicotinsäure und Nicotinamid auch Verbindungen mit Vitamin B-Eigenschaften verstanden werden, wie z. B. Adenin, Cholin, Pantothensäure, Biotin, Adenylsäure, Folsäure, Orotsäure, Pangamsäure, Carnitin, p-Aminobenzoesäure, myo-Inosit und Liponsäure sowie Vitamin C, Vitamine der D-Gruppe, E-Gruppe, F-Gruppe, H-Gruppe, I- und J-Gruppe, K-Gruppe und P-Gruppe. Zu Wirkstoffen im Sinne der Erfindung gehören auch Peptidtherapeutika. Zu Pflanzenbehandlungsmitteln zählen z. B. Vinclozolin, Epoxiconazol und Quinmerac.

Das erfindungsgemäße Bindemittel ist beispielsweise zur Verarbeitung mit folgenden Wirkstoffen geeignet:

Acebutolol, Acetylcystein, Acetylsalicylsäure, Aciclovir, Alprazolam, Alfacalcidol, Allantoin, Allopurinol, Ambroxol, Amikacin, Amilorid, Aminoessigsäure, Amiodaron, Amitriptylin, Amlodipin, Amoxicillin, Ampicillin, Ascorbinsäure, Aspartam, Astemizol, Atenolol, Beclomethason, Benserazid, Benzalkonium-Hydrochlorid, Benzocain, Benzoesäure, Betamethason, Bezafibrat, Biotin, Biperiden, Bisoprolol, Bromazepam, Bromhexin, Bromocriptin, Budesonid, Bufexamac, Buflomedil, Buspiron, Coffein, Campher, Captopril, Carbamazepin, Carbidopa, Carboplatin, Cefachlor, Cefalexin, Cefadroxil, Cefazolin, Cefixim, Cefotaxim, Ceftazidim, Ceftriaxon, Cefuroxim, Selegilin, Chloramphenicol, Chlorhexidin, Chlor-pheniramin, Chlortalidon, Cholin, Cyclosporin, Cilastatin, Cimetidin, Ciprofloxacin, Cisapride, Cisplatin, Clarithromycin, Clävulansäure, Clomipramin, Clonazepam, Clonidin, Clotrimazol, Codein, Cholestyramin, Cromoglycinsäure, Cyanocobalamin, Cyproteron, Desogestrel, Dexamethason, Dexpanthenol, Dextromethorphan, Dextropropoxiphen, Diazepam, Diclofenac, Digoxin, Dihydrocodein, Dihydroergotamin, Dihydroergotoxin, Diltiazem, Diphenhydramin, Dipyridamol, Dipyron, Disopyramid, Domperidon, Dopamin, Doxycyclin, Enalapril, Ephedrin, Epinephrin, Ergocalciferol, Ergotamin, Erythromycin, Estradiol, Ethinylestradiol, Etoposid, Eucalyptus globulus, Famotidin, Felodipin, Fenofibrat, Fenoterol, Fentanyl, Flavin-Mononucleotid, Fluconazol, Flunarizin, Fluorouracil, Fluoxetin, Flurbiprofen, Furosemid, Gallopamil, Gemfibrozil, Gentamicin, Gingko biloba, Glibenclamid, Glipizid, Clozapin, Glycyrrhiza glabra, Griseofulvin, Guaifenesin, Haloperidol, Heparin, Hyaluronsäure, Hydrochlorothiazid, Hydrocodon, Hydrocortison, Hydromorphon, Ipratropium-Hydroxid, Ibuprofen, Imipenem, Indomethacin, Iohexol, Iopamidol, Isosorbid-Dinitrat, Isosorbid-Mononitrat, Isotretinoin, Itraconazol, Ketotifen, Ketoconazol, Ketoprofen, Ketorolac, Labetalol, Lactulose, Lecithin, Levocarnitin, Levodopa, Levoglutamid, Levonorgestrel, Levothyroxin, Lidocain, Lipase, Imipramin, Lisinopril, Loperamid, Lorazepam, Lovastatin, Medroxyprogesteron, Menthol, Methotrexat, Methyldopa, Methylprednisolon, Metoclopramid, Metoprolol, Miconazol, Midazolam, Minocyclin, Minoxidil, Misoprostol, Morphin, Multivitamin-Mischungen bzw. -Kombinationen und Mineralsalze, N-Methylephedrin, Naftidrofuryl, Naproxen, Neomycin, Nicardipin, Nicergolin, Nicotinamid, Nicotin, Nicotinsäure, Nifedipin, Nimodipin, Nitrazepain, Nitrendipin, Nizatidin, Norethisteron, Norfloxacin, Norgestrel, Nortriptylin, Nystatin, Ofloxacin, Omeprazol, Ondansetron, Pancreatin, Panthenol, Pantothensäure, Paracetamol, Penicillin G, Penicillin V, Phenobarbital, Pentoxifyllin, Phenoxymethylpenicillin, Phenylephrin, Phenylpropanolamin, Phenytoin, Piroxicam, Polymyxin B, Povidon-Iod, Pravastatin, Prazepam, Prazosin, Prednisolon, Prednison, Bromocriptin, Propafenon, Propranolol, Proxyphyllin, Pseudoephedrin, Pyridoxin, Quinidin, Ramipril, Ranitidin, Reserpin, Retinol, Riboflavin, Rifampicin, Rutosid, Saccharin, Salbutamol, Salcatonin, Salicylsäure, Simvastatin, Somatropin, Sotalol, Spironolacton, Sucralfat, Sulbactam, Sulfamethoxazol, Sulfasalazin, Sulpirid, Tamoxifen, Tegafur, Teprenon, Terazosin, Terbutalin, Terfenadin, Tetracyclin, Theophyllin, Thiamin, Ticlopidin, Timolol, Tranexainsäure, Tretinoin, Triamcinolon-Acetonid, Triamteren, Trimethoprim, Troxerutin, Uracil, Valproinsäure, Vancomycin, Verapamil, Vitamin E, Folinsäure, Zidovudin.

Bevorzugte Wirkstoffe sind Ibuprofen (als Racemat, Enantiomer oder angereichertes Enantiomer), Ketoprofen, Flurbiprofen, Acetylsalicylsäure, Verapamil, Paracetamol, Nifedipin oder Captopril.

Zur Herstellung der festen Dosierungsformen wird ein plastisches Gemisch der Komponenten (Schmelze) bereitgestellt, das anschließend einem Formgebungsschritt unterzogen wird. Das Vermischen der Komponenten und die Bildung der Schmelze können auf unterschiedliche Weise erfolgen. Das Vermischen kann vor, während und/oder nach der Bildung der Schmelze erfolgen. Beispielsweise können die Komponenten zuerst vermischt und dann aufgeschmolzen oder gleichzeitig vermischt und aufgeschmolzen werden. Häufig erfolgt noch eine Homogenisierung des plastischen Gemisches, um eine hochdisperse Verteilung des Wirkstoffes zu erhalten.

Insbesondere bei Verwendung von empfindlichen Wirkstoffen hat es sich aber als bevorzugt erwiesen, zuerst das polymere Bindemittel, gegebenenfalls zusammen mit üblichen pharmazeutischen Additiven, aufzuschmelzen und vorzuvermischen und dann den (die) empfindlichen Wirkstoff(e) in "Intensivmischern" in plastischer Phase bei sehr kleinen Verweilzeiten einzumischen (Homogenisieren). Der (die) Wirkstoff(e) kann (können) dabei in fester Form oder als Lösung oder Dispersion eingesetzt werden.

Im Allgemeinen werden die Komponenten als solche in das Herstellungsverfahren eingesetzt. Sie können jedoch auch in flüssiger Form, d. h. als Lösung, Suspension oder Dispersion zur Anwendung kommen.

Als Lösungsmittel für die flüssige Form der Komponenten kommt in erster Linie Wasser oder ein mit Wasser mischbares, organisches Lösungsmittel oder ein Gemisch davon mit Wasser in Betracht. Brauchbare Lösungsmittel sind aber auch mit Wasser nicht mischbare oder mischbare, organische Lösungsmittel. Geeignete, mit Wasser mischbare Lösungsmittel sind insbesondere $C_1$-$C_4$-Alkanole, wie Ethanol, Isopropanol oder n-Propanol, Polyole, wie Ethylenglykol, Glycerin und Polyethylenglykole. Geeignete, mit Wasser nicht mischbare Lösungsmittel sind Alkane, wie Pentan oder Hexan, Ester, wie Ethylacetat oder Butylacetat, chlorierte Kohlenwasserstoffe, wie Methylenchlorid und aromatische Kohlenwasserstoffe, wie Toluol und Xylol. Ein weiteres brauchbares Lösungsmittel ist flüssiges $CO_2$.

Welches Lösungsmittel im Einzelfall verwendet wird, hängt von der aufzunehmenden Komponente und deren Eigenschaften ab. Beispielsweise kommen pharmazeutische Wirkstoffe häufig in Form eines Salzes, das im Allgemeinen wasserlöslich ist, zur Anwendung. Wasserlösliche Wirkstoffe können daher als wässrige Lösung eingesetzt werden oder vorzugsweise in die wässrige Lösung oder Dispersion des Bindemittels aufgenommen werden. Entsprechendes gilt für Wirkstoffe, die in einem der genannten Lösungsmittel löslich sind, wenn die flüssige Form der zur Anwendung kommenden Komponenten auf einem organischen Lösungsmittel basiert.

Gegebenenfalls kann an die Stelle des Aufschmelzens ein Lösen, Suspendieren oder Dispergieren in den

oben genannten Lösungsmitteln, falls erwünscht und/oder erforderlich unter Zusatz geeigneter Hilfsstoffe, wie z. B. Emulgatoren, treten. Das Lösungsmittel wird dann im Allgemeinen unter Bildung der Schmelze in einer geeigneten Apparatur, z. B. einem Extruder, entfernt. Im Folgenden soll dies von dem Begriff Vermischen umfasst werden.

Das Aufschmelzen und/oder Vermischen erfolgt in einer für diesen Zweck üblichen Vorrichtung. Besonders geeignet sind Extruder oder gegebenenfalls beheizbare Behälter mit Rührwerk, z. B. Kneter, (wie der unten noch erwähnten Art).

Als Mischapparat sind insbesondere solche Vorrichtungen brauchbar, die in der Kunststofftechnologie zum Mischen eingesetzt werden. Geeignete Vorrichtungen sind beispielsweise beschrieben in "Mischen beim Herstellen und Verarbeiten von Kunststoffen", H. Pahl, VDI-Verlag, 1986. Besonders geeignete Mischapparaturen sind Extruder und dynamische und statische Mischer, sowie Rührkessel, einwellige Rührwerke mit Abstreifvorrichtungen, insbesondere sogenannte Pastenrührwerke, mehrwellige Rührwerke, insbesondere PDSM-Mischer, Feststoffmischer sowie vorzugsweise MischKnetreaktoren (z. B. ORP, CRP, AP, DTB der Firma List oder Reactotherm der Firma Krauss-Maffei oder Ko-Kneter der Fa. Buss), Doppelmuldenkneter (Trogmischer) und Stempelkneter (Innenmischer) oder Rotor/Stator-Systeme (z. B. Dispax der Firma IKA).

Bei empfindlichen Wirkstoffen erfolgt vorzugsweise zunächst das Aufschmelzen des polymeren Bindemittels in einem Extruder und anschließend das Zumischen des Wirkstoffs in einem Misch-Knetreaktor. Bei weniger empfindlichen Wirkstoffen kann man dagegen zum intensiven Dispergieren des Wirkstoffs ein Rotor/Stator-System einsetzen.

Das Beschicken der Mischvorrichtung erfolgt je nach deren Konzeption kontinuierlich oder diskontinuierlich in üblicher Weise. Pulverförmige Komponenten können im freien Zulauf, z. B. über eine Differentialdosierwaage eingeführt werden. Plastische Massen können direkt aus einem Extruder eingespeist oder über eine Zahnradpumpe, die insbesondere bei hohen Viskositäten und hohen Drüken von Vorteil ist, zugespeist werden. Flüssige Medien können über ein geeignetes Pumpenaggregat zudosiert werden.

Das durch Vermischen und/oder Aufschmelzen des Bindemittels, des Wirkstoffes und gegebenenfalls des Additivs oder der Additive erhaltene Gemisch ist teigig bis zähflüssig (thermoplastisch) oder flüssig und daher extrudierbar. Die Glasübergangstemperatur des Gemisches liegt unter der Zersetzungstemperatur aller in dem Gemisch enthaltenen Komponenten. Das Bindemittel soll vorzugsweise in physiologischer Umgebung löslich oder quellbar sein.

Die Verfahrensschritte Vermischen und Aufschmelzen können in derselben Apparatur oder in zwei oder mehreren getrennt arbeitenden Vorrichtungen ausgeführt werden. Die Zubereitung einer Vormischung kann in einer der oben beschriebenen üblichen Mischvorrichtungen durchgeführt werden. Eine solche Vormischung kann dann direkt, z. B. in einen Extruder, eingespeist und anschließend ggf. unter Zusatz weiterer Komponenten extrudiert werden.

Das erfindungsgemäße Verfahren erlaubt es, als Extruder Einschneckenmaschinen, kämmende Schnekkenmaschinen oder auch Mehrwellextruder, insbesondere Zweischnecken-Extruder, gleichsinnig oder gegensinnig drehend und gegebenenfalls mit Knetscheiben ausgerüstet, einzusetzen. Wenn bei der Extrusion ein Lösungsmittel verdampft werden muss, sind die Extruder im Allgemeinen mit einem Verdampfungsteil ausgerüstet. Besonders bevorzugt sind Extruder der ZKS-Baureihe von Werner u. Pfleiderer.

Erfindungsgemäß können auch mehrschichtige pharmazeutische Formen durch Koextrusion hergestellt werden, wobei mehrere Gemische aus den oben beschriebenen Komponenten bei der Extrusion so in einem Werkzeug zusammengeführt werden, dass sich der gewünschte Schichtaufbau der mehrschichtigen pharmazeutischen Form ergibt. Vorzugsweise verwendet man verschiedene Bindemittel für verschiedene Schichten.

Mehrschichtige Arzneiformen umfassen vorzugsweise zwei oder drei Schichten. Sie können in offener oder geschlossener Form vorliegen, insbesondere als offene oder geschlossene Mehrschichttabletten.

Wenigstens eine der Schichten enthält wenigstens einen pharmazeutischen Wirkstoff. Es ist auch möglich, einen weiteren Wirkstoff in eine andere Schicht aufzunehmen. Dies hat den Vorteil, dass zwei miteinander unverträgliche Wirkstoffe verarbeitet werden können oder dass die Freisetzungscharakteristik des Wirkstoffes gesteuert werden kann.

Das Ausformen erfolgt durch Koextrusion, wobei die Gemische aus den einzelnen Extrudern oder anderen Aggregaten in ein gemeinsames Koextrusionswerkzeug geführt und ausgetragen werden. Die Form der Koextrusionswerkzeuge richtet sich nach der gewünschten pharmazeutischen Form. Beispielsweise sind Werkzeuge mit ebenem Austrittsspalt, sogenannte Breitschlitzwerkzeuge, und Werkzeuge mit kreisringspaltförmigem Austrittsquerschnitt geeignet. Die Düsenauslegung erfolgt dabei in Abhängigkeit von dem zur Anwendung kommenden polymeren Bindemittel und der gewünschten pharmazeutischen Form.

Das erhaltene Gemisch ist vorzugsweise lösungsmittelfrei, d. h. es enthält weder Wasser noch ein organisches Lösungsmittel.

Das plastische Gemisch wird in der Regel einer abschließenden Formgebung unterzogen. Dabei kann eine Vielzahl von Formen, je nach Werkzeug und Art der Formung, erzeugt werden. Beispielsweise lässt sich bei Verwendung eines Extruders der extrudierte Strang zwischen einem Band und einer Walze, zwischen zwei Bändern oder zwischen zwei Walzen, wie in der EP-A-358 105 beschrieben, oder durch Kalandrierung in einem Kalander mit zwei Formwalzen, siehe beispielsweise EP-A-240 904, formen. Durch Extrusion und Heiß- oder Kaltabschlag des Stranges können weitere Formen erhalten werden, beispielsweise kleinteilige und gleichmäßig geformte Granulate. Die Heißgranulierung führt in der Regel zu linsenförmigen Dosierungsformen (Tabletten) mit einem Durchmesser von 1 bis 10 mm, während die Kaltgranulierung normalerweise zu zylinderförmigen Produkten mit einem Verhältnis von Länge zu Durchmesser von 1 bis 10 und einem Durchmesser von 0,5 bis 10 mm führt. So können einschichtige, bei Anwendung der Koextrusion aber auch offene oder geschlossene, mehrschichtige Dosierungsformen hergestellt werden, beispielsweise Oblongtabletten, Dragees, Pastillen und Pellets. Die erhaltenen Granulate können anschließend auch zu Pulver gemahlen und in üblicher Weise zu Tabletten verpresst werden. Mikropastillen können durch das Rotoform-Sandvik-Verfahren hergestellt werden. Diese Dosierungsformen können in einem nachgeschalteten Verfahrensschritt nach üblichen Methoden gerundet und/oder mit einem Coating versehen werden. Geeignete Materialien für Filmüberzüge sind z. B. Polyacrylate, wie die Eudragit-Typen, Celluloseester, wie die Hydroxypropylcellulosephthalate, sowie Celluloseether, wie Ethylcellulose, Hydroxypropylmethylcellulose oder Hydroxypropylcellulose.

Im Einzelnen kann es zur Ausbildung von festen Lösungen kommen. Der Begriff "feste Lösungen" ist dem Fachmann geläufig, beispielsweise aus der eingangs zitierten Literatur. In festen Lösungen von Wirkstoffen in Polymeren liegt der Wirkstoff molekulardispers im Polymer vor.

Die folgenden Beispiele sollen das erfindungsgemäße Verfahren veranschaulichen, ohne es jedoch zu beschränken. Die Kalandrierung der extrudierten Schmelze erfolgte wie in der EP-A-240 904 beschrieben.

Beispiele

Die Extrusion erfolgte jeweils mit einem Zweischneckenextruder ZKS 30 der Firma Werner u. Pfleiderer mit 5 Schüssen unter den in dem jeweiligen Beispiel angegebenen Bedingungen.

Beispiel 1

520 g Copolymer aus 50 Gew.-% Vinylpyrrolidon und 50 Gew.-% Methylmethacrylat (K-Wert 42; 1 %ig in Aceton) wurden mit 480 g Verapamil-Hydrochlorid extrudiert und zu 650 mg-Oblong-Tabletten kalandriert.

Die Extrusion erfolgte unter folgenden Bedingungen:

| | |
|---|---|
| Schuss 1 | 61 °C |
| Schuss 2 | 90 °C |
| Schuss 3 | 120 °C |
| Schuss 4 | 113 °C |
| Schuss 5 | 113 °C |
| Düse | 110 °C |

Die Freisetzung des Wirkstoffs aus den Tabletten wurde nach dem Paddle-Modell (USP, pH change) untersucht. Sie betrug nach 1 Stunde 18 %, nach 2 Stunden 26 % und nach 8 Stunden 41 %.

Beispiel 2

500 g Copolymer aus 50 Gew.-% Vinylpyrrolidon und 50 Gew.-% Methylmethacrylat (K-Wert 42; 1 %ig in Aceton) werden mit 500 g Vinclozolin extrudiert, gekühlt und granuliert.

Die Extrusion erfolgte unter folgenden Bedingungen:

| | |
|---|---|
| Schuss 1 | 60 °C |
| Schuss 2 | 96 °C |
| Schuss 3 | 130 °C |

(fortgesetzt)

| | |
|---|---|
| Schuss 4 | 135 °C |
| Schuss 5 | 120 °C |
| Düse | 110 °C |

Es wurde ein transparentes, röntgenamorphes Granulat erhalten.

Beispiel 3

760 g Copolymer aus 50 Gew.-% Vinylpyrrolidon und 50 Gew.-% Methylmethacrylat (K-Wert 46,2; 1 %ig in Aceton) wurden mit 240 g Nifedipin extrudiert und zu 250 mg-Oblong-Tabletten kalandriert.

Die Extrusion erfolgte unter folgenden Bedingungen:

| | |
|---|---|
| Schuss 1 | 77 °C |
| Schuss 2 | 126 °C |
| Schuss 3 | 160 °C |
| Schuss 4 | 149 °C |
| Schuss 5 | 147 °C |
| Düse | 141 °C |

Die Freisetzung des Wirkstoffes aus den Tabletten wurden nach dem Paddle-Modell (USP, Phosphatpuffer + 0,1 % SDS; pH 6,8; 100 Upm) untersucht Sie betrug nach 1 Stunde 2 %, nach 2 Stunden 4 % und nach 8 Stunden 9 %.

Beispiel 4

600 g Copolymer aus 50 Gew.-% Vinylpyrrolidon und 50 Gew.-% Methylmethacrylat (K-Wert 41; 1 %ig in Aceton) wurden mit 250 g Kollidon VA 64 (Polyvinylpyrrolidon) und 120 g Nifedipin extrudiert und zu 500 mg-Oblong-Tabletten kalandriert.

Die Extrusion erfolgte unter folgenden Bedingungen:

| | |
|---|---|
| Schuss 1 | 120 °C |
| Schuss 2 | 154 °C |
| Schuss 3 | 160 °C |
| Schuss 4 | 157 °C |
| Schuss 5 | 166 °C |
| Düse | 151 °C |

Die Freisetzung des Wirkstoffes aus den Tabletten wurde nach dem Paddle-Modell (USP, Phosphatpuffer + 0,1 % SDS, pH 6,8, 100 Upm) untersucht. Sie betrug nach 1 Stunde 2 %, nach 2 Stunden 4 % und nach 8 Stunden 12 %.

Beispiel 5

300 g Copolymer aus 50 Gew.-% Vinylpyrrolidon und 50 Gew.-% Methylmethacrylat (K-Wert 43; 1 %ig in Aceton) wurden mit 580 g Kollidon VA 64 (Polyvinylpyrrollindon) und 120 g Nifedipin extrudiert und zu 500 mg-Oblong-Tabletten kalandriert.

Die Extrusion erfolgte unter folgenden Bedingungen:

| | |
|---|---|
| Schuss 1 | 119 °C |
| schuss 2 | 145 °C |
| Schuss 3 | 151 °C |
| Schuss 4 | 146 °C |
| Schuss 5 | 145 °C |
| Düse | 145 °C |

Die Freisetzung des Wirkstoffs aus den Tabletten wurde nach dem Paddle-Modell (Phosphatpuffer + 0,1 % SDS, pH 6,8, 100 Upm) untersucht. Sie betrug nach 1 Stunde 5 %, nach 2 Stunden 9 %, und nach 8 Stunden 31 %.

Beispiel 6

150 g Copolymer aus 50 Gew.-% Vinylpyrrolidon und 50 Gew.-% Methylmathacrylat (K-Wert 43; 1 %ig in Aceton) wurden mit 730 g Kollidon VA 64 (Polyvinylpyrrolidon) und 120 g Nifedipin extrudiert und zu 500 mg-Oblong-Tabletten kalandriert.

Die Extrusion erfolgte unter folgenden Bedingungen:

| | |
|---|---|
| Schuss 1 | 113 °C |
| Schuss 2 | 139 °C |
| Schuss 3 | 146 °C |
| Schuss 4 | 142 °C |
| Schuss 5 | 140 °C |
| Düse | 145 °C |

Die Freisetzung des Wirkstoffs aus den Tabletten wurde nach dem Paddle-Modell (USP, Phosphatpuffer + 0,1 % SDS, pH 6,8, 100 Upm) untersucht. Sie betrug nach 1 Stunde 10 %, nach 2 Stunden 18 % und nach 8 Stunden 63 %.

Beispiel 7

150 g Copolymer aus 50 Gew.-% Vinylpyrrolidon und 50 Gew.-% Methylmethacrylat (K-Wert 32,4; 1 %ig in Aceton) wurden mit 30 g Klucel EF (Hydroxypropylcellulose) und 288 g Verapamil-Hydrochlorid extrudiert und zu 500 mg-Oblong-Tabletten kalandriert.

Die Extrusion erfolgte unter folgenden Bedingungen:

| | |
|---|---|
| Schuss 1 | 23 °C |
| Schuss 2 | 47 °C |
| Schuss 3 | 90 °C |
| Schuss 4 | 131 °C |
| Schuss 5 | 113 °C |

(fortgesetzt)

| | |
|---|---|
| Düse | 96 °C |

Die Freisetzung des Wirkstoffs aus den Tabletten wurde nach dem Paddle-Modell (USP, pH change) untersucht. Sie betrug nach 1 Stunde 26 % nach 2 Stunden 38 % und nach 8 Stunden 65 %.

Beispiel 8

520 g Copolymer aus 30 Gew.-% Vinylpyrrolidon und 70 Gew.-% Methylmethacrylat (K-Wert 42; 1 %ig in Aceton) wurden mit 480 g Verapamil-Hydrochlorid extrudiert und zu 650 mg-Oblong-Tabletten kalandriert.
Die Extrusion erfolgte unter folgenden Bedingungen:

| | |
|---|---|
| Schuss 1 | 60 °C |
| Schuss 2 | 85 °C |
| Schuss 3 | 115 °C |
| Schuss 4 | 110 °C |
| Schuss 5 | 110 °C |
| Düse | 110 °C |

Die Freisetzung des Wirkstoffs aus den Tabletten wurde nach dem Paddle-Modell (USP, pH change) untersucht. Sie betrug nach 1 Stunde 17 %, nach 2 Stunden 24 % und nach 8 Stunden 39%.

Beispiel 9

160 mg Copolymer aus 50 Gew.-% Vinylpyrrolidon und 50 Gew.-% Methylmethacrylat (K-Wert 31,9; 1 %ig in Ethanol) wurden mit 3,4 mg Aerosil 200, 1,6 mg Magnesiumstearat und 160 mg Propranolol-Hydrochlorid mit einer Preßkraft von 18 kN zu einer facettierten 10 mm Tablette direkttablettiert.
Die Freisetzung des Wirkstoffs aus den Tabletten wurde nach dem Paddle-Modell (USP, pH 7,4) untersucht. Sie betrug nach 1 stunde 33 %, nach 2 Stunden 56 % und nach 8 Stunden 95 %.

Vergleichsbeispiel 1

500 g Copolymer aus 60 Gew.-% N-Vinylpyrrolidon und 40 Gew.-% Vinylacetat werden mit 500 g Verapamil-Hydrochlorid unter den nachfolgend angegebenen Bedingungen extrudiert und zu 500 mg-Oblong-Tabletten kalandriert.
Die Extrusion erfolgte unter folgenden Bedingungen:

| | |
|---|---|
| Schuss 1 | 90 °C |
| Schuss 2 | 100 °C |
| Schuss 3 | 110 °C |
| Schuss 4 | 120 °C |
| Schuss 5 | 130 °C |
| Düse | 135 °C |

Die Freisetzung des Wirkstoffs aus den Tabletten wurde nach dem Paddle-Modell (USP, pH change) untersucht. Sie betrug nach 3 Stunden 100 %.

Vergleichsbeispiel 2

500 g Copolymer aus 30 Gew.-% Vinylpyrrolidon und 70 Gew.-% Vinylacetat werden mit 500 g Verapamil-Hydrochlorid unter den nachfolgend angegebenen. Bedingungen extrudiert und zu 500 mg-Oblong-Tabletten kalandriert.

Die Extrusion erfolgte unter folgenden Bedingungen:

| | |
|---|---|
| Schuss 1 | 30 °C |
| Schuss 2 | 60 °C |
| Schuss 3 | 100 °C |
| Schuss 4 | 100 °C |
| Schuss 5 | 120 °C |
| Düse | 120 °C |

Die Freisetzung des Wirkstoffs aus den Tabletten wurde nach dem Paddle-Modell (USP, pH change) untersucht. Sie betrug nach 8 Stunden 100 %.

Bestimmung der Wasseraufnahme

Zur Bestimmung der Wasseraufnahme wurden N-Vinylpyrrolidon/Methylmethacrylat-Copolymere mit unterschiedlichen Monomerenzusammensetzungen (siehe Tabelle 1) in Form dünner Filme 24 Stunden zum Quellen in Wasser gelegt. Die Wasseraufnahme wurde durch Wiegen ermittelt und nach der angegebenen Formel in Prozentwerte umgerechnet.

Tabelle 1

| Bsp. Nr. | Copolymer | Monomeren-verhältnis [Gew.-Teile] | Einwaage trocken [g] | Gewicht nach Quellung [g] | Wasseraufnahme [1] [Gew.-%] |
|---|---|---|---|---|---|
| B 10 | NVP/MMA | 50:50 | 0,96 | 1,38 | 44 |
| B 11 | NVP/MMA | 40:60 | 0,81 | 1,17 | 44 |
| B 12 | NVP/MMA | 30:70 | 1,22 | 1,60 | 31 |
| B 13 | NVP/MMA | 25:75 | 1,10 | 1,42 | 29 |

1) $$\frac{\text{Gew. hydratisiertes Bindemittel} - \text{Gew. trockenes Bindemittel}}{\text{Gew. trockenes Bindemittel}} \cdot 100\ \%$$

**Patentansprüche**

**1.** Feste Dosierungsform, enthaltend wenigstens ein polymeres Bindemittel, wenigstens einen Wirkstoff und gegebenenfalls übliche Additive, dadurch gekennzeichnet, dass das polymere Bindemittel

a) 10 bis 60 Gew.-% mindestens eines N-Vinyllactams der Formel I

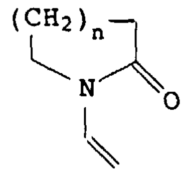

(I)

worin n für 1, 2 oder 3 steht, und

b) 40 bis 90 Gew.-% Methylmethacrylat

einpolymerisiert enthält und eine Wasseraufnahme durch Quellen von weniger als 50 Gew.-%, bezogen auf das Gewicht des Bindemittels, aufweist.

2. Feste Dosierungsform nach Anspruch 1, dadurch gekennzeichnet, dass das polymere Bindemittel

a) 20 bis 55 Gew.-% mindestens eines eines N-Vinyllactams der Formel I und

b) 45 bis 80 Gew.-% Methylmethacrylat

einpolymerisiert enthält.

3. Feste Dosierungsform nach einem der Ansprüche 1 oder 2, dadurch gekennzeichnet, dass das polymere Bindemittel eine Wasseraufnahme durch Quellen von weniger als 40 Gew.-%, bezogen auf das Gewicht des Bindemittels, aufweist.

4. Feste Dosierungsform nach einem der vorhergehenden Ansprüche, dadurch gekennzeichnet, dass das N-Vinyllactam N-Vinyl-Pyrrolidon ist.

5. Feste Dosierungsform nach einem der vorhergehenden Ansprüche, dadurch gekennzeichnet, dass das polymere Bindemittel zusätzlich 0,1 bis 2,0 Gew.-% mindestens eines vernetzenden Monomers einpolymerisiert enthält.

6. Feste Dosierungsform nach einem der vorhergehenden Ansprüche, dadurch gekennzeichnet, dass das Copolymer außerdem mindestens ein weiteres Monomer einpolymerisiert enthält, das ausgewählt ist unter Vinylestern aliphatischer $C_1$-$C_{22}$-Carbonsäuren, $\alpha,\beta$-ethylenisch ungesättigten $C_3$-$C_8$-Mono- und Dicarbonsäuren, deren Anhydriden, Estern und Halbestern mit $C_1$-$C_8$-Alkanolen, Estern und Halbestern mit $C_1$-$C_4$-Diolen oder Di- ($C_1$-$C_4$)-Alkylamino-$C_1$-$C_4$-alkanolen, Amiden und Nitrilen dieser Carbonsäuren, $\alpha,\beta$-ethylenisch ungesättigten Sulfonsäuren und den Salzen oder quaternisierten Produkten davon.

7. Feste Dosierungsform nach einem der vorhergehenden Ansprüche in Form von pharmazeutischen Mitteln, Pflanzenbehandlungsmitteln, Futtermittelzusatzstoffen und Nahrungsmittelzusätzen.

8. Verfahren zur Herstellung der festen Dosierungsformen gemäß einem der vorhergehenden Ansprüche, dadurch gekennzeichnet, dass man die Komponenten gewünschtenfalls zunächst aufschmilzt oder direkt mischt und das erhaltene plastische Gemisch zur gewünschten Dosierungsform formt.